# EUROPEAN PATENT SPECIFICATION

(11) **EP 1 545 511 B1**
(45) Date of publication and mention of the grant of the patent: **09.05.2007**
(21) Application number: 03750659.9
(22) Date of filing: 26.09.2003
(51) Int. Cl.: A61K 31/4045, A61P 25/00

(54) **COMBINED USE OF METHYLPHENIDATE AND MELATONIN FOR TREATING ATTENTION-DEFICIT HYPERACTIVE DISORDER**
KOMBINIERTE VERWENDUNG VON MELATONIN UND METHYLPHENIDAT ZUR BEHANDLUNG VON AUFMERKSAMKEITS-/HYPERAKTIVITÄTSSTÖRUNGEN.
UTILISATION COMBINEE DE METHYLPHENIDATE ET DE MELATONINE POUR LE TRAITEMENT D'UN TROUBLE D'HYPERACTIVITE AVEC DEFICIENCE DE L'ATTENTION

(30) Priority: 26.09.2002 EP 02021810
(43) Date of publication of application: 29.06.2005
(73) Proprietor: Pooger Properties Limited, Jersey, Channel Islands JE1 4HH (GB)
(72) Inventor: KRUISINGA, Roelof, Johannes, Hendrik, NL-2243 HW Wassenaar (NL)
(74) Representative: Huygens, Arthur Victor
(86) International application number: PCT/EP2003/010827
(87) International publication number: WO 2004/028532

(56) References cited:
- WO-A-02/076452
- HOBAN T F: "SLEEPLESSNESS IN CHILDREN WITH NEURODEVELOPMENTAL DISORDERS EPIDEMIOLOGY AND MANAGEMENT" CNS DRUGS, ADIS INTERNATIONAL, AUCKLAND, NZ, vol. 14, no. 1, July 2000 (2000-07), pages 11-22, XP001013140 ISSN: 1172-7047
- KIRBY KELLY ET AL: "DIAGNOSIS AND MANAGEMENT OF ATTENTION-DEFICIT/HYPERACTIVITY DISORDER IN CHILDREN" CURRENT OPINION IN PEDIATRICS, CURRENT SCIENCE, PHILADELPHIA, PA, US, vol. 13, no. 2, 2001, pages 190-199, XP001013123 ISSN: 1040-8703
- CECIL ET AL: "Melatonin for treatment of sleeping disorders in children with attention deficit hyperactivity disorder." EUR.J.PEDRIATR., vol. 162, 2003, pages 554-555, XP002269839 3RD JUNE 2003

## Description

The present invention relates a to a combination of of methylphenidate and melatonin for the treatment of attention-deficit hyperactivity disorder ("ADHD") in mammals, including humans. The invention relates also to a pharmaceutical composition comprising methylphenidate and melatonin.

As described in EP-A-0 896 536, ADHD is a condition affecting a significant proportion of children and which is manifest by learning difficulties, restlessness, inability to settle to any task, argumentativeness, low frustration tolerance and aggressive conduct. A traditional method of treating such children was by administration of psychostimulant such as methylphenidate.

Methylphenidate, also known under the trademark Ritalin®, is a medication prescribed for individuals (usually children) who have an abnormally high level of activity or attention-deficit hyperactivity disorder (ADHD). According to the U.S. National Institute of Mental Health, about 3 to 5 percent of the general population in the U.S.A. has the disorder, which is characterized by agitated behavior and an inability to focus on tasks. Methylphenidate also is occasionally prescribed for treating narcolepsy.

Methylphenidate is a central nervous system (CNS) stimulant. It has effects similar to, but more potent than, caffeine and less potent than amphetamines. It has a notably calming effect on hyperactive children and a "focusing" effect on those with ADHD.

Recent research at Brookhaven National Laboratory may begin to explain how methylphenidate helps people with ADHD. The researchers used positron emission tomography (PET - a noninvasive brain scan) to confirm that administering normal therapeutic doses of methylphenidate to healthy, adult men increased their dopamine levels. The researchers speculate that methylphenidate amplifies the release of dopamine, a neurotransmitter, thereby improving attention and focus in individuals who have dopamine signals that are weak, such as individuals with ADHD. See, N. Volkow et al., *of Neuroscience* (2001) 21:RC121:1-5.

While psychostimulants are useful in increasing attention spans, they have major side-effects, including loss of appetite and insomnia and do not deal with the problems of hyperactivity.

The aforementioned EP-A-0 896 536 discloses the use of lofexidine, 2-[α-(2,6-dichlorophenoxy)ethyl-Δ²-imidazole, in the manufacture of a medicament for treating ADHD, which reportedly does not incur the same level of side-effects as clonidine. The latter compound (see Hunt *et al*., Journal of the American Academy of Child Adolescent Psychiatry 24 (1995)) has been shown to be effective in treating ADHD, but it may also cause hypotension and a high level of sedation as a side-effect. It is stated in said EP reference that while a measure of sedation can be useful in the treatment of hyperactive children, it does not assist in increasing attention span.

Melatonin (N-acetyl-5-methoxytryptamine) is an endogenous hormone of the pineal gland, a small organ (approx. 100 mg) located in the mid-brain above the third ventricle (A.B. Lerner *et al., J. Amer. Chem. Soc.* 1958; 80:2587). The rate-limiting enzyme for its synthesis, N-acetyltransferase (NAT) is produced only during the night. Night-time values of NAT are more than 100-fold greater than daytime levels. Melatonin is also produced by extra-pineal tissues, that lightens skin color in amphibians by reversing the darkening effect of MSH (melanotropin). Melatonin has been postulated as the mediator of photic-induced anti-gonadotropic activity in photoperiodic mammals and has also been shown to be involved in thermoregulation in some ectotherms and in affecting locomotor activity rhythms in sparrows.

Melatonin, when used experimentally, is synthesised chemically and has been studied extensively in clinical and preclinical trials to examine the effects of the circadian SCN clock (A.J. Lewy *et al., Behav. Brain Res.* 1996; 73:1-2 131-4).

Non-prepublished international patent application PCT/EP02/03317 discloses that melatonin has usefulness in the treatment of ADHD, and provides the use of at least one of melatonin, a melatonin analogue, or a pharmaceutically acceptable salt of melatonin or said melatonin analogue, in the preparation of a medicament for the treatment of ADHD in mammals, in particular human beings.

The present invention is based on the surprising finding that the combined use of methylphenidate and melatonin has a beneficial effect on the treatment of ADHD, which will frequently exceed the effect of the individual active compounds.

As used hereinafter, unless stated otherwise, the term "melatonin" includes melatonin per se, a melatonin analogue, i.e. a substance exhibiting high affinity for melatonin receptors, or a pharmaceutically acceptable salt of melatonin or a melatonin analogue.

The medicament for the treatment of ADHD comprising the combination of methylphenidate and melatonin as active ingredients is suitably administered to the mammal in the form of a pharmaceutical composition. The administration may be by way of oral or parenteral administration.

The medicament can be administered in conventional form for oral administration, e.g. as tablets, lozenges, dragees and capsules. However, for the administration of the drug to children, which is likely to be its major use, it may be preferred to formulate the composition as an oral liquid preparation such as a syrup, a nasal spray, or a suppository. The medicament can also be administered parenterally, e.g. by intramuscular or subcutaneous injection, using formulations in which the medicament is employed in a saline or other pharmaceutically acceptable, injectable composition.

An amount effective to treat the disorder hereinbefore described depends on the usual factors such as the nature and severity of the disorder being treated, the weight of the mammal, the specific compounds of choice, and considerations and preferences of the prescriber. The amount of active ingredients to be administered usually will be in the range of nanograms to 50 mg or more per dose. However, a unit dose will normally contain 1 to 1000 mg, suitably 1 to 500 mg, for example an amount in the range of from 2 to 400 mg such as 2, 5, 10, 20, 30, 40, 50, 100, 200, 300 and 400 mg of the active ingredient. Unit doses will normally be administered once or more than once per day, for example 1, 2, 3, 4, 5 or 6 times a day, more usually 1 to 4 times a day, such that the total daily dose is normally in the range, for a 70 kg adult, of 1 to 1000 mg, for example 1 to 500 mg, that is in the range of approximately 0.01 to 15 mg/kg/day, more usually 0.1 to 6 mg/kg/day, for example 1 to 6 mg/kg/day.

It is greatly preferred that the combination of methylphenidate and melatonin according to the invention is administered in the form of a unit-dose composition, such as a unit dose oral, such as sub-lingual, rectal, topical or parenteral (especially intravenous) composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use. The preparation of such compositions is well known to people skilled in the art and can be optimized in a routine way without exerting inventive skill and without undue experimentation.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include, mannitol and other similar agents. Suitable disintegrants include starch derivatives such as sodium starch glycollate. Suitable lubricants include, for example, magnesium stearate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral formulations further include controlled release formulations which may also be useful in the practice of this invention. The controlled release formulation may be designed to give an initial high dose of the active material and then a steady dose over an extended period of time, or a slow build up to the desired dose rate, or variations of these procedures. Controlled release formulations also include conventional sustained release formulations, for example tablets or granules having an enteric coating.

Nasal spray compositions are also a useful way of administering the pharmaceutical preparations of this invention to patients such as children for whom compliance is difficult. Such formulations are generally aqueous and are packaged in a nasal spray applicator which delivers a fine spray of the composition to the nasal passages.

Suppositories are also a traditionally good way of administering drugs to children and can be used for the purposes of this invention. Typical bases for formulating suppositories include water-soluble diluents such as polyalkylene glycols and fats, e.g. cocoa oil and polyglycol ester or mixtures of such materials.

For parenteral administration, fluid unit dose forms are prepared containing the active compounds and a sterile vehicle. The active compounds, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle.

Parenteral suspensions are prepared in substantially the same manner except that the active compounds are suspended in the vehicle instead of being dissolved and sterilised usually by exposure to ethylene oxide before suspending in the sterile vehicle. Advantageously, a surfactant or wetting agent is included in the composition to facilitate uniform distribution of the combined active compounds of the invention.

As is common practice, the compositions will usually be accompanied by written or printed directions for use in the medical treatment concerned.

The present invention further provides a pharmaceutical composition comprising methylphenidate and at least one of melatonin, a melatonin analogue, or a pharmaceutically acceptable salt of melatonin or said melatonin analogue, and a pharmaceutically acceptable carrier. These pharmaceutical compositions may be prepared in a manner known per se or in the manner as hereinbefore described.

In the treatment of ADHD patients in accordance with the invention, methylphenidate and melatonin can be used simultaneously or subsequently, preferably melatonin after methylphenidate in the latter case, optionally together with other active materials. The latter materials are preferably chosen such that either their activity is enhanced, preferably in a synergistic way, or undesired side-effects are suppressed by melatonin. For example, melatonin which can be used in conjunction with another active ingredient may additionally contain, besides methylphenidate, one or more substances selected from the group of stimulants, hormones, analogues of such hormones, phyto-hormones, analogues of such phyto-hormones like phyto-estrogen, and anti-oxidants like phyto-vitamins C and E, flavonoids.

The recommended dosages of methylphenidate are well-documented in the art. For example, suitable dosages of methylphenidate which is usually but not exclusively administered as its chloride, include unit-dosages of about 10 mg. The dosage is individual and depends inter alia on the indication. For example, the average dose in the treatment of narcolepsia usually is a tablet of 10 mg twice or three times a day. As another example, the dosage to treat hyperkinetic syndrome with children from the age of about 6 years starts with about 0.25 mg per kg body weight orally per day (for example 5 mg in the morning and in the afternoon = ½ tablet). This dose may be doubled weekly up to about 2 mg per kg body weight per day, but usually not exceeding 60 mg per day.

Preliminary investigations show the following dose rates for melatonin. For the occasional self-treatment of mild insomnia in adults: 0.3 to 3 mg oral or sublingual dosage (PO), in the evening hours approximately 1 to 2 hours before habitual bedtime. May take up to 6 mg PO if needed. For the adjunctive treatment of insomnia related to major depression: Adults: 5 to 10 mg oral extended release formulations (PO) taken 1 to 2 hours prior to habitual bedtime. In one 4-week placebo-controlled study of 19 patients with major depressive disorder treated with fluoxetine, the sub-group of 10 patients who received concomitant slow-release melatonin at 9 pm for sleep reported significantly improved sleep quality scores versus the patients receiving fluoxetine alone. Melatonin treatment avoided the need for additional sleep medications. No differences in the rates of improvement of depressive symptoms or side effects were reported between the two groups. (Dolberg et al; 1998)

For the treatment of delayed sleep phase syndrome resulting from circadian rhythm disruption, including patients with autism, blindness, Rett's syndrome, or developmental disabilities in adults: Doses of 5 to 7 mg oral immediate release formulations (PO) once daily at bedtime have been used in the blind to entrain circadian rhythms to a 24-hour day. (Sack et al; 1991); in children: Doses of 2.5 to 7.5 mg PO once daily before expected bedtime have been used. The average onset of sleep occurred within 1 hour of melatonin administration. Most children were on concomitant anticonvulsant therapies. Melatonin was administered nightly for up to 4 weeks and appeared to be well tolerated.

In the Netherlands melatonin is an "over de counter" hormone product as in the USA, which is to be sold without restrictions in dosages ranging from 0.1 to 0.3 mg. Melatonin is supplied in various grades. Toxic effects have not been reported. At a dose of 240 mg retarded responses have been described (H.R. Lieberman, et al., *Brain Research* (1984) 323:201-207).

ADHD is a behaviour disorder with concomitant insomnia in 54% of the cases (D. Efron et al., *Pediatrics* (1997) 100(4) 662-666. In treating ADHD patients with methyl phenidate the problems relating to insomnia have increased to 64% (A.R. Adesman et al., *Pediatrics Clinics North America* (1999) 46(5):945-963. Some authors attribute ADHD behaviour to insufficient sleep (M. Thunström, *Acta Pediatrica* (2002) 91:584-592). The most plausible hypothesis for a pervasive ADHD-syndrome is likely to be found in a disturbed transfer of stimuli (at noradrenergous/serotenergous level) at various locations in the brain (inter alia at prefrontal, stratial, and even cerebellum levels). See, M. Mercugliano, *Pediatric Clinics of North America* (1999) 46:5 831-843.

In a pilot study it was already demonstrated that melatonin 3 mg gave a significant reduction in the time for insomnia for patients who had been previously treated with methyl phenidate (C.V. Tjon Pian Gi et al., poster presentation, *Annual Scientific Meeting European Society for Pediatric Research,* September 2002.

It has now been found that pure melatonin 2.5 mg is active as a sleep-inducing agent to ADHD patients with insomnia disorders who had been first treated with methyl phenidate.

The present invention is therefore further characterised in that methyl phenidate and melatonin are applied together, i.e. simultaneously or subsequently, in treating ADHD in mammals, in particular humans and more in particular children. It is preferred that melatonin is administered following the administration of methyl phenidate.

The amounts of methylphenidate and melatonin may be decreased individually as compared with the normal individual use of these active ingredients to reach a similar or even better effect by the combined use than with the individual components.

Although the invention has been described primarily as a therapy for children, it can also be used for adults, although dosage rates may be different in the case of adults. Adaptation and optimization of dosages can be readily achieved by skilled persons without undue experimentation.

## Claims

1. Use of methylphenidate and at least one of melatonin, a melatonin analogue, or one or more pharmaceutically acceptable salts or esters thereof, in the preparation of a medicament for the treatment of ADHD in a mammal, especially a human being.

2. Use as claimed in claim 1 wherein the melatonin or melatonin analogue is employed in an amount of from 0.005 to 1.00 mg/kg in treating ADHD.

3. Use as claimed in any one of the preceding claims wherein the medicament is formulated as a controlled release preparation.

4. Use as claimed in any one of the preceding claims wherein the medicament is formulated as a solid oral formulation.

5. Use as claimed in any one of the preceding claims wherein the medicament additionally contains one or more substances selected from the group of stimulants, hormones, analogues of such hormones, phyto-hormones, analogues of such phyto-hormones, and anti-oxidants.

6. A pharmaceutical composition comprising, as active ingredients, methylphenidate and at least one of melatonin, a melatonin analogue, or one or more pharmaceutically acceptable salts or esters thereof, in conjunction with a pharmaceutically acceptable carrier.

7. A pharmaceutical composition according to claim 6 comprising, as active ingredients, methylphenidate and melatonin, in conjunction with a pharmaceutically acceptable carrier.

8. Use of methylphenidate and at least one of melatonin, a melatonin analogue, or one or more pharmaceutically acceptable salts or esters thereof as claimed in claim 1, wherein methylphenidate and at least one of melatonin, a melatonin analogue, or one or more pharmaceutically acceptable salts or esters thereof are comprised in separate forms of administration.

## Patentansprüche

1. Verwendung von Methylphenidat und mindestens eines Melatonins, eines Melatoninanalogons oder eines pharmazeutisch verträglichen Salzes oder Esters von Melatonin oder des Melatoninanalogons zur Herstellung eines Medikaments zur Behandlung des ADHD in einem Säuger, insbesondere einem Menschen.

2. Verwendung nach Anspruch 1, wobei das Melatonin oder Melatoninanalogon in einer Menge von 0,005 bis 1,00 mg/kg bei der Behandlung von ADHD verwendet wird.

3. Verwendung nach einem der vorstehenden Ansprüche, wobei das Medikament als ein Mittel zur kontrollierten Freisetzung formuliert ist.

4. Verwendung nach einem der vorstehenden Ansprüche, wobei das Medikament als eine feste Oralformulierung formuliert ist.

5. Verwendung nach einem der vorstehenden Ansprüche, wobei das Medikament zusätzlich eine oder mehrere Substanzen enthält, die aus Stimulantien, Hormonen, Analogasolcher Hormone, Phytohormonen, Analoga solcher Phytohormone and Antioxidationsmitteln ausgewählt sind.

6. Pharmazeutische Zusammensetzung, die als aktive Bestandteile Methylphenidat und mindestens eines Melatonins, eines Melatoninanalogons oder eines pharmazeutisch verträglichen Salzes von Melatonin oder des Melatoninanalogons aufweisen, in Konjunktion mit einem pharmazeutischen akzeptierbaren Träger.

7. Pharmazeutische Zusammensetzung nach Anspruch 6, die als aktive Bestandteile Methylphenidat und Melatonin aufweisen, in Konjunktion mit einem pharmazeutischen akzeptierbaren Träger.

8. Verwendung von Methylphenidat und mindestens eines Melatonins, eines Melatoninanalogons oder eines pharmazeutisch verträglichen Salzes oder Esters von Melatonin oder des Melatoninanalogons nach Anspruch 1, wobei Methylphenidat und mindestens eines Melatonins, eines Melatoninanalogons oder eines pharmazeutisch verträglichen Salzes von Melatonin oder des Melatoninanalogons in separate Verabreichungsformen aufgenommen sind.

## Revendications

1. Utilisation de méthylphénidate et au moins un composé parmi la mélatonine, un analogue de mélatonine, ou un ou plusieurs sels ou esters pharmaceutiquement acceptables de mélatonine ou dudit analogue de mélatonine, dans la préparation d'un médicament destiné au traitement de l'ADHD chez un mammifère, en particulier chez un être humain.

2. Utilisation selon la revendication 1 dans laquelle la mélatonine ou l'analogue de mélatonine est employé en une quantité de 0,005 à 1,00 mg/kg pour traiter l'ADHD.

3. Utilisation selon l'une quelconque des revendications précédentes dans laquelle le médicament est formulé en préparation à libération contrôlée.

4. Utilisation selon l'une quelconque des revendications précédentes dans laquelle le médicament est formulé en formulation orale solide.

5. Utilisation selon l'une quelconque des revendications précédentes dans laquelle le médicament contient en plus une ou plusieurs substances choisies dans le groupe des stimulants, des hormones, des analogues de ces hormones, des phytohormones, des analogues de ces phytohormones et des antioxydants.

6. Composition pharmaceutique comprenant, comme ingrédients actives, méthylphénidate et au moins un composé parmi la mélatonine, un analogue de mélatonine, ou un ou plusieurs sels ou esters pharmaceutiquement acceptables de mélatonine ou dudit analogue de mélatonine, en liaison avec un support pharmaceutiquement acceptable.

7. Composition pharmaceutique selon la revendication 6, comprenant, comme ingrédients actives, méthylphénidate et mélatonine, en liaison avec un support pharmaceutiquement acceptable.

8. Utilisation de méthylphénidate et au moins un composé parmi la mélatonine, un analogue de mélatonine, ou un ou plusieurs sels ou esters pharmaceutiquement acceptables de mélatonine ou dudit analogue de mélatonine selon la revendication 1, dans laquelle méthylphénidate et au moins un composé parmi la mélatonine, un analogue de mélatonine, ou un ou plusieurs sels ou esters pharmaceutiquement acceptables de mélatonine ou dudit analogue de mélatonine sont compris sous formes séparées d'administration.
